# EUROPEAN PATENT APPLICATION

(11) **EP 4 497 819 A1**
(43) Date of publication of application: **29.01.2025**
(21) Application number: 23774917.1
(22) Date of filing: 22.03.2023
(51) Int. Cl.: C12N 1/21, C12N 15/31, C12P 7/42

(54) **GENETICALLY-MODIFIED MICROORGANISM FOR PRODUCING 3-HYDROXYADIPIC ACID AND/OR 3-OXOADIPIC ACID AND PRODUCTION METHOD FOR SAID CHEMICAL PRODUCTS**

(30) Priority: 23.03.2022 JP 2022046640
(71) Applicant: Toray Industries, Inc., Tokyo 103-8666 (JP)
(72) Inventor: ISOBE, Kyohei, Kamakura-shi, Kanagawa 248-8555 (JP); KAWAMURA, Kenji, Kamakura-shi, Kanagawa 248-8555 (JP); YAMADA, Katsushige, Kamakura-shi, Kanagawa 248-8555 (JP)
(74) Representative: Kador & Partner Part mbB
(86) International application number: PCT/JP2023/011072
(87) International publication number: WO 2023/182322

(57) **Abstract**

Disclosed is a genetically modified microorganism that can produce 3-hydroxyadipic acid and/or 3-oxoadipic acid in high productivity, and a method of producing 3-hydroxyadipic acid and/or 3-oxoadipic acid using the genetically modified microorganism. The genetically modified microorganism is a microorganism having an ability to produce 3-hydroxyadipic acid and/or 3-oxoadipic acid, in which the activity of an enzyme that catalyzes the reaction to generate 3-oxoadipyl-CoA and CoA from succinyl-CoA and acetyl-CoA is enhanced, and the expression level of an organophosphate efflux transporter or a homolog thereof is increased.

## Description

### TECHNICAL FIELD

The present invention relates to a genetically modified microorganism that produces 3-hydroxyadipic acid and/or 3-oxoadipic acid in high yields, and a method of producing 3-hydroxyadipic acid and/or 3-oxoadipic acid using the genetically modified microorganism.

### BACKGROUND ART

3-hydroxyadipic acid (IUPAC name: 3-hydroxyhexanedioic acid) is a dicarboxylic acid containing six carbon atoms. These can be used as a raw material for the production of polyesters by polymerization with polyols or as a raw material for the production of polyamides by polymerization with polyamines. Additionally, compounds obtained by adding ammonia to the end of these converting the resultants to lactams can also be used as raw materials for polyamides.

3-oxoadipic acid (also called β-ketoadipic acid, IUPAC name: 3-oxohexanedioic acid) is a dicarboxylic acid containing six carbon atoms and having a molecular weight of 160.12. 3-oxoadipic acid can be used as a raw material for the production of polyesters by polymerization with polyols or as a raw material for the production of polyamides by polymerization with polyamines. Additionally, 3-oxoadipic acid can also be used alone as a raw material for polyamides by adding ammonia to the end of 3-oxoadipic acid and converting the resultant to lactams.

As documents related to production of 3-hydroxyadipic acid or 3-oxoadipic acid using microorganisms, Patent Document 1 discloses a method of producing 3-hydroxyadipic acid using a *Serratia* microorganism having enhanced enzyme activity for catalyzing the reaction from succinyl-CoA and acetyl-CoA to 3-oxoadipyl-CoA and CoA, and Patent Document 2 reports that 3-oxoadipic acid (3-oxoadipate) can be generated as an intermediate in an adipic acid biosynthetic pathway during a process in which adipic acid is produced by a non-naturally occurring microorganism using succinyl-CoA and acetyl-CoA as starting raw materials.

Methods of producing chemical products using microorganisms involve utilizing an efflux transporter (transporter) that discharges the chemical products to be produced from the cells (e.g., Patent Document 3, and Non-Patent Documents 1 and 2). Patent Document 4 describes a method of producing 3-hydroxyadipic acid and/or α-hydromuconic acid by using a genetically modified microorganism having functional deficiency or deterioration of a dicarboxylic acid efflux transporter. The document describes the biosynthetic pathway for these substances as passing through an enzymatic reaction that converts succinyl-CoA and acetyl-CoA into 3-oxoadipyl-CoA and CoA, and the functional deficiency or deterioration of a dicarboxylic acid efflux transporter resulting in improved productivity of these substances.

### PRIOR ART DOCUMENTS

### PATENT DOCUMENTS

Patent Document 1: WO 2017/209102
Patent Document 2: WO 2009/151728
Patent Document 3: JP 2017-528126 A
Patent Document 4: WO 2021/187533

### NON-PATENT DOCUMENTS

Non-Patent Document 1: J. Ind. Microbiol Biotechnol. 2012 Apr; 39(4): 641-7
Non-Patent Document 2: Pesticide Biochemistry and Physiology 140(2017) 65-68

### SUMMARY OF THE INVENTION

### PROBLEMS TO BE SOLVED BY THE INVENTION

The present invention aims to create a genetically modified microorganism that can produce 3-hydroxyadipic acid and/or 3-oxoadipic acid in high yields, and a method of producing 3-hydroxyadipic acid and/or 3-oxoadipic acid using the genetically modified microorganism.

### MEANS FOR SOLVING THE PROBLEMS

The present inventor has intensively studied in order to achieve the object described above and consequently found that in a microorganism having an ability to produce 3-hydroxyadipic acid and/or 3-oxoadipic acid, the expression level of an efflux transporter for an organophosphate compound that is a substance completely unrelated to 3-hydroxyadipic acid and/or 3-oxoadipic acid can be increased to improve the productivity for 3-hydroxyadipic acid and/or 3-oxoadipic acid, thereby completing the present invention.

That is, the present invention provides the following (1) to (13):
(1) A genetically modified microorganism having an ability to produce 3-hydroxyadipic acid and/or 3-oxoadipic acid,
   wherein the activity of an enzyme that catalyzes the reaction to generate 3-oxoadipyl-CoA and CoA from succinyl-CoA and acetyl-CoA is enhanced, and
   wherein the expression level of an organophosphate efflux transporter is increased.
(2) The genetically modified microorganism of (1), wherein the organophosphate efflux transporter is a phosphoserine efflux transporter and/or a glyphosate efflux transporter.
(3) The genetically modified microorganism of (1) or (2), wherein the organophosphate efflux transporter is YhhS, MdtD, RhtB, MFS40, or a homolog thereof.
(4) The genetically modified microorganism of any one of (1) to (3), wherein the expression level of the organophosphate efflux transporter is increased by introducing a gene encoding any of the polypeptides (A) to (C) below.
   (A) a polypeptide consisting of the amino acid sequence of SEQ ID NO: 1, 3, 28-30, 46, 48, or 50;
   (B) a polypeptide having a sequence identity of 60% or more to the amino acid sequence of SEQ ID NO: 1, 3, 28-30, 46, 48, or 50, and having a function to improve the productivity of 3-hydroxyadipic acid and/or 3-oxoadipic acid; and
   (C) a polypeptide consisting of the same amino acid sequence as the amino acid sequence of SEQ ID NO: 1, 3, 28-30, 46, 48, or 50 except that one or several amino acids are replaced, deleted, inserted and/or added, and having a function to improve the productivity of 3-hydroxyadipic acid and/or 3-oxoadipic acid.
(5) The genetically modified microorganism of any one of (1) to (4), wherein the activity(ies) of enzyme(s) that catalyze(s) the reaction to reduce 3-oxoadipyl-CoA to generate 3-hydroxyadipyl-CoA and/or the reaction to generate 3-hydroxyadipic acid from 3-hydroxyadipyl-CoA is/are further enhanced.
(6) The genetically modified microorganism of any one of (1) to (5), wherein the activity of an enzyme that catalyzes the reaction to generate 3-oxoadipic acid from 3-oxoadipyl-CoA is further enhanced.
(7) The genetically modified microorganism of any one of (1) to (6), wherein the microorganism is a microorganism belonging to the genus *Escherichia* or *Serratia.*
(8) A method of producing 3-hydroxyadipic acid and/or 3-oxoadipic acid, comprising the step of culturing the genetically modified microorganism of any one of (1) to (7).
(9) A method of producing a genetically modified microorganism, comprising the steps of:
   genetically modifying a microorganism having an ability to produce 3-hydroxyadipic acid and/or 3-oxoadipic acid to enhance the activity of an enzyme that catalyzes the reaction to generate 3-oxoadipyl-CoA and CoA from succinyl-CoA and acetyl-CoA; and
   increasing the expression level of YhhS or a homolog thereof in the microorganism.
(10) A method of producing 3-hydroxyadipic acid and/or 3-oxoadipic acid, comprising the step of culturing a microorganism having an ability to produce 3-hydroxyadipic acid and/or 3-oxoadipic acid and having increased expression level of an organophosphate efflux transporter.
(11) A method of producing adipic acid, comprising the step A of producing 3-hydroxyadipic acid and/or 3-oxoadipic acid by the method of (8) or (10); and the step B (hydrogenation step) of reacting the 3-hydroxyadipic acid and/or 3-oxoadipic acid with hydrogen in the presence of a hydrogenation catalyst.
(12) A method of producing a polyamide, comprising:
   the step of producing adipic acid according to the method of claim 11, and
   the step C of polycondensing the adipic acid and a diamine.
(13) The method of producing a polyamide of claim 11, wherein the diamine is a diamine containing 1,4-butanediamine, 1,5-pentanediamine, or hexamethylenediamine.

### EFFECT OF THE INVENTION

The genetically modified microorganism of the present invention can produce 3-hydroxyadipic acid and/or 3-oxoadipic acid in higher yields than the parent microorganism strain whose expression level of an organophosphate efflux transporter is not increased.

### DETAILED DESCRIPTION OF THE INVENTION

Hereinafter, 3-hydroxyadipic acid may be abbreviated as 3HA, and 3-oxoadipic acid as 3OA. The gene encoding YhhS may be referred to as yhhS. The gene encoding MdtD may be referred to as mdtD. The gene encoding RhtB may be referred to as rhtB. The gene encoding EmrD may be referred to as emrD. In addition, 3-oxoadipyl-CoA may be abbreviated as 3OA-CoA, 3-hydroxyadipyl-CoA may be abbreviated as 3HA-CoA, and coenzyme A may be abbreviated as CoA. In addition, a nucleic acid encoding a functional polypeptide may be referred to as a gene.

The reaction scheme 1 described below shows exemplary reaction pathways for production of 3HA and/or 3OA by microorganisms. In this scheme, the reaction A represents a reaction to generate 3-oxoadipyl-CoA and CoA from acetyl-CoA and succinyl-CoA. The reaction B represents a reaction that reduces 3-oxoadipyl-CoA to generate 3-hydroxyadipyl-CoA. The reaction C represents a reaction to generate 3-hydroxyadipic acid from 3-hydroxyadipyl-CoA. The reaction D represents a reaction to generate 3-oxoadipic acid from 3-oxoadipyl-CoA. Microorganisms having an ability to produce 3HA and/or 3OA are known to have an enzyme that catalyzes at least the reaction A of these reaction pathways (see WO2019/107516).

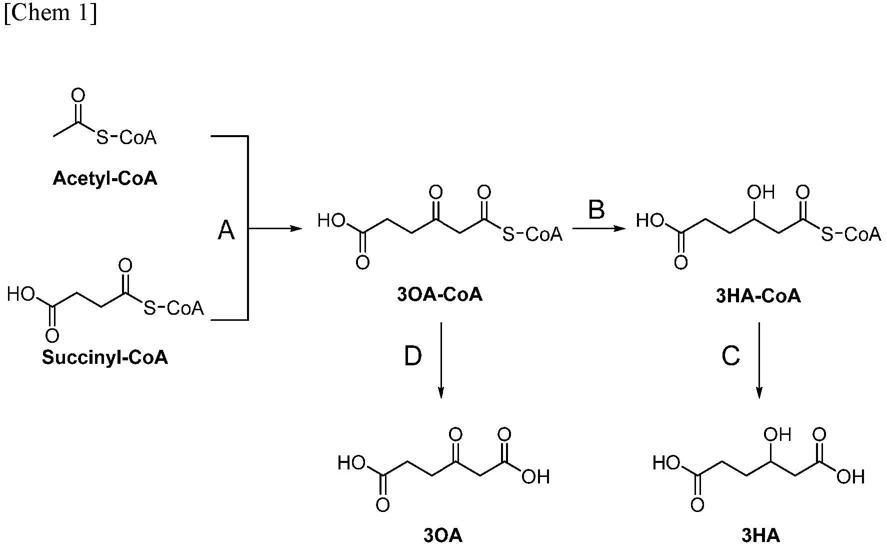

The genetically modified microorganism of the present invention is a microorganism having an ability to produce 3HA and/or 3OA, wherein the enzyme activity for the reaction to generate 3-oxoadipyl-CoA and CoA from acetyl-CoA and succinyl-CoA (reaction A) is enhanced, and the expression level of an organophosphate efflux transporter is increased, thereby improving the productivity for 3HA and/or 3OA by the microorganism having an ability to produce 3HA and/or 3OA as its technical feature. The term "genetic modification" means artificially modifying genes.

Organophosphate compounds refer to compounds having a structure in which an organic compound such as an alcohol, a carboxylic acid, or an amino acid and phosphoric acid. Specific examples thereof include phosphoserine produced by ester bonding of serine which is an amino acid having a hydroxy group, to phosphoric acid, and glyphosate that is glycine with its nitrogen atom substituted with a phosphonomethyl group.

Organophosphate efflux transporters are membrane proteins having an activity to allow intracellular organophosphate compounds to efflux to the outside of the cells. Increasing the expression level of an organophosphate efflux transporter means increasing the expression level of a protein that has the organophosphate efflux function and is originally possessed by microorganisms, and/or making microorganisms express a protein that has the organophosphate efflux function and is not originally possessed by the microorganisms. Preferred and specific examples of organophosphate efflux transporters whose expression level is to be increased in the present invention include phosphoserine efflux transporters and/or glyphosate efflux transporters.

Specific examples of phosphoserine efflux transporters include YhhS, MdtD, and RhtB proteins, and homologs thereof described in JP 2017-528126 A as O-phosphoserine efflux transporters.

Specific examples of glyphosate efflux transporters include YhhS protein and homologs thereof described in J. Ind. Microbiol Biotechnol. 2012 Apr; 39(4): 641-7*,* and MFS40 protein and homologs thereof described in Pesticide Biochemistry and Physiology 140 (2017) 65-68*.*

The documents cited for description of the specific examples of phosphoserine efflux transporters and glyphosate efflux transporters have no description that organophosphate efflux transporters exemplified by YhhS and the like have an activity as a dicarboxylic acid efflux transporter. Thus, increased expression level of an organophosphate efflux transporter resulting in improved ability to produce 3HA and/or 3OA that are dicarboxylic acids containing six carbon atoms as shown in Examples herein means that the organophosphate efflux transporter functions as an efflux transporter of 3HA and/or 3OA. It is an unexpected finding for those skilled in the art that organophosphate efflux transporters also function as efflux transporters for 3HA and/or 3OA.

Hereinafter, YhhS, MdtD, RhtB, and MFS40, and homologs thereof that are suitable as organophosphate efflux transporters whose expression level is increased in the present invention will be described in detail.

YhhS is a protein that is presumed to be a major facilitator superfamily (MFS)-type transporter. A specific example thereof is YhhS derived from *Escherichia coli* str. K-12 substr. MG1655 (NCBI Protein ID: NP_417930, SEQ ID NO: 1). An example of the yhhS gene encoding the YhhS protein is yhhS derived from *Escherichia coli* str. K-12 substr. MG1655 (NCBI Gene ID: 947982, SEQ ID NO: 2). Proteins having the same amino acid sequence as SEQ ID NO: 1 except that one or several amino acids are replaced, deleted, inserted, and/or added, and having a function to improve the productivity of 3HA and/or 3OA when its expression level is increased can also be used as YhhS. Here, the range of "one or several" is preferably within 10, more preferably within 5, still more preferably within 4, particularly preferably 1 or within 2. Proteins having an amino acid sequence with a sequence identity (the definition is described later) to the amino acid sequence of SEQ ID NO: 1 of 60% or more, preferably 70% or more, more preferably 80% or more, still more preferably 90% or more, still more preferably 95% or more, still more preferably 97% or more, still more preferably 98% or more, still more preferably 99% or more, and having a function to improve the productivity of 3HA and/or 3OA when its expression level is increased can also be used as YhhS.

Homologs of YhhS are proteins that have an amino acid sequence with high sequence identity to YhhS and are presumed to have a similar function or structure as YhhS.

Homologs of YhhS can be readily obtained, for example, by performing BLAST (Basic Local Alignment Search Tool) search using the amino acid sequence of YhhS as a query sequence on public database such as NCBI (National Center for Biotechnology Information) or KEGG (Kyoto Encyclopedia of Genes and Genomes), and referring to the genes encoding matching sequences. Homologs of YhhS can also be obtained by PCR using oligonucleotides prepared based on the nucleotide sequence of a gene encoding YhhS or a homolog thereof as primers and genomic DNA from another organism as a template.

Homologs of YhhS are proteins that have an amino acid sequence with high sequence identity to YhhS and are presumed to have a similar function or structure as YhhS, and those having a sequence identity to the amino acid sequence of YhhS derived from *Escherichia coli* str. K-12 substr. MG1655 (NCBI Protein ID: NP_417930, SEQ ID NO: 1) of specifically 60% or more, preferably 70% or more, more preferably 80% or more, still more preferably 90% or more, particularly preferably 95% or more, and having a function to improve the productivity of 3HA and/or 3OA when its expression level is increased can be used. Specific examples of such YhhS homologs include a YhhS homolog derived from *Serratia grimesii* strain NBRC13537 (SEQ ID NO: 3), a YhhS homolog derived from *Salmonella enterica* strain ATCC9150 (NCBI Protein ID: AAV79243, SEQ ID NO: 28), a YhhS homolog derived from *Klebsiella pneumoniae* strain MGH78578 (NCBI Protein ID: ABR79226, SEQ ID NO: 29), and a YhhS homolog derived from *Pectobacterium carotovorum* strain PC1 (NCBI Protein ID: ACT11150, SEQ ID NO: 30). Examples of genes encoding these proteins include a yhhS homolog derived from *Serratia grimesii* strain NBRC13537 (SEQ ID NO: 4), a yhhS homolog derived from *Salmonella enterica* strain ATCC9150 (NCBI Gene ID: CP000026, REGION:3539049..3540266, SEQ ID NO: 31), a yhhS homolog derived from *Klebsiella pneumoniae* strain MGH78578 (NCBI Gene ID: CP000647, REGION:4195189..4196409, SEQ ID NO: 32), and a yhhS homolog derived from *Pectobacterium carotovorum* strain PC1 (NCBI Gene ID: CP001657, REGION:113478..114677, SEQ ID NO: 33). As used herein and in claims, the term "sequence identity" means percentage of the same amino acids or bases in an optimal alignment of two amino acid sequences or nucleotide sequences aligned with or without gap introduction relative to the entire overlapping amino acid sequence (comprising the amino acid as the translation start site) or nucleotide sequence (comprising the start codon), and is calculated according to the equation (1). In the equation (1), the length of the shorter sequence to be compared is 100 amino acids or more, or 300 bases or more. If the length is less than 100 amino acid or less than 300 bases, the sequence identity is not defined. The sequence identity can be readily investigated, for example, in BLAST using default parameters. The sequence identity can also be investigated using similar functions available in software such as Genetyx. The sequence identities between the amino acid sequences of SEQ ID NOs: 1, 28, 29, 30, and 3 are calculated according to the equation (1) using the function (%Identity Matrix) in Genetyx to be 90.9, 84.4, 70.2, and 61.4%, respectively. Sequence identity (%) = the number of matchings (gaps are not counted)/length of shorter sequence (length without gaps at both ends) × 100

MdtD (also called YegB) is a protein belonging to the major facilitator superfamily (MFS) and called Putative multidrug resistance protein. A specific example thereof is MdtD derived from *Escherichia coli* str. K-12 substr. MG1655 (NCBI Protein ID: NP_416581, SEQ ID NO: 46). An examples of genes encoding MdtD is mdtD derived from *Escherichia coli* str. K-12 substr. MG1655 (NCBI Gene ID: 946601, SEQ ID NO: 47). Proteins having the same amino acid sequence as SEQ ID NO: 46 except that one or several amino acids are replaced, deleted, inserted, and/or added, and having a function to improve the productivity of 3HA and/or 3OA when its expression level is increased can also be used as MdtD. Here, the range of "one or several" is preferably within 10, more preferably within 5, still more preferably within 4, particularly preferably 1 or within 2. Proteins having an amino acid sequence with a sequence identity to the amino acid sequence of SEQ ID NO: 46 of 60% or more, preferably 70% or more, more preferably 80% or more, still more preferably 90% or more, still more preferably 95% or more, still more preferably 97% or more, still more preferably 98% or more, still more preferably 99% or more, and having a function to improve the productivity of 3HA and/or 3OA when its expression level is increased can also be used as MdtD.

RhtB (also called YigK) is a protein called Homoserine/homoserine lactone efflux protein. A specific example thereof is RhtB derived from *Escherichia coli* str. K-12 substr. MG1655 (NCBI Protein ID: YP_026265, SEQ ID NO: 48). An example of genes encoding RhtB is RhtB derived from *Escherichia coli* str. K-12 substr. MG1655 (NCBI Gene ID: 948316, SEQ ID NO: 49). Proteins having the same amino acid sequence as SEQ ID NO: 48 except that one or several amino acids are replaced, deleted, inserted, and/or added, and having a function to improve the productivity of 3HA and/or 3OA when its expression level is increased can also be used as RhtB. Here, the range of "one or several" is preferably within 10, more preferably within 5, still more preferably within 4, particularly preferably 1 or within 2. Proteins having an amino acid sequence with a sequence identity to the amino acid sequence of SEQ ID NO: 48 of 60% or more, preferably 70% or more, more preferably 80% or more, still more preferably 90% or more, still more preferably 95% or more, still more preferably 97% or more, still more preferably 98% or more, still more preferably 99% or more, and having a function to improve the productivity of 3HA and/or 3OA when its expression level is increased can also be used as RhtB.

MFS40 is a protein belonging to the major facilitator superfamily (MFS). A specific example thereof is MFS40 derived from *Aspergillus oryzae* strain RIB40 (NCBI Protein ID: XP_001826887, SEQ ID NO: 50). An example of genes encoding MFS40 is the MFS40 gene derived from *Aspergillus oryzae* strain RIB40 (NCBI Reference Sequence: XM_001826835.2, SEQ ID NO: 51). Proteins having the same amino acid sequence as SEQ ID NO: 50 except that one or several amino acids are replaced, deleted, inserted, and/or added, and having a function to improve the productivity of 3HA and/or 3OA when its expression level is increased can also be used as MFS40. Here, the range of "one or several" is preferably within 10, more preferably within 5, still more preferably within 4, particularly preferably 1 or within 2. Proteins having an amino acid sequence with a sequence identity to the amino acid sequence of SEQ ID NO: 50 of 60% or more, preferably 70% or more, more preferably 80% or more, still more preferably 90% or more, still more preferably 95% or more, still more preferably 97% or more, still more preferably 98% or more, still more preferably 99% or more, and having a function to improve the productivity of 3HA and/or 3OA when its expression level is increased can also be used as MFS40.

Homologs of MdtD, RhtB, and MFS40 are proteins that have amino acid sequences with high sequence identities to the proteins and are presumed to have similar functions or structures as the proteins. Homologs with sequence identities relative to MdtD derived from *Escherichia coli* str. K-12 substr. MG1655 (NCBI Protein ID: NP_416581, SEQ ID NO: 46), RhtB derived from *Escherichia coli* str. K-12 substr. MG1655 (NCBI Protein ID: YP_026265, SEQ ID NO: 48), or MFS40 derived from *Aspergillus oryzae* strain RIB40 (NCBI Protein ID: XP_001826887, SEQ ID NO: 50) of specifically 60% or more, preferably 70% or more, more preferably 80% or more, still more preferably 90% or more, particularly preferably 95% or more, and having a function to improve the productivity of 3HA and/or 3OA when its expression level is increased can be used. These homologs can be readily obtained, for example, by performing BLAST (Basic Local Alignment Search Tool) search using the amino acid sequence of MdtD as a query sequence on public database such as NCBI (National Center for Biotechnology Information) or KEGG (Kyoto Encyclopedia of Genes and Genomes), and referring to the genes encoding matching sequences. Homologs of MdtD can also be obtained by PCR using oligonucleotides prepared based on the nucleotide sequence of a gene encoding MdtD or a homolog thereof as primers and genomic DNA from another organism as a template.

It is preferred that the above-described proteins suitable as organophosphate efflux transporters be expressed by introducing the gene encoding the protein into a microorganism host and expressing the gene therein as an exogenous gene (see Examples below).

The method itself of enhancing the enzyme activity for the reaction to generate 3-oxoadipyl-CoA and CoA from acetyl-CoA and succinyl-CoA (reaction A) in a microorganism having an ability to produce 3HA and/or 3OA is known, and the enhancement can be preferably performed, for example, according to the method described in WO2017/209102.

Examples of enzymes having an activity to catalyze the reaction to generate 3-oxoadipylCoA and CoA from succinyl-CoA and acetyl-CoA include enzymes such as acetyl-CoA acetyltransferase, β-ketoacyl CoA acyltransferase, 3-oxoadipyl-CoA acyltransferase, β-ketoadipyl-CoA acyltransferase, acetyl-CoA C-acetyltransferase, acetoacetyl-CoA thiolase, beta-acetoacetyl coenzyme A thiolase, 2-methylacetoacetyl-CoA thiolase, 3-oxothiolase, acetyl coenzyme A thiolase, acetyl-CoA acetyltransferase, acetyl-CoA:N-acetyltransferase, acetyl-CoA C-acyltransferase, beta-ketothiolase, 3-ketoacyl-CoA thiolase, beta-ketoacyl coenzyme A thiolase, beta-ketoacyl-CoA thiolase, beta-ketoadipyl coenzyme A thiolase, beta-ketoadipyl-CoA thiolase, 3-ketoacyl coenzyme A thiolase, 3-ketoacyl thiolase, 3-ketothiolase, 3-oxoacyl-CoA thiolase, 3-oxoacyl-coenzyme A thiolase, 6-oxoacyl-CoA thiolase, acetoacetyl-CoA beta-ketothiolase, acetyl-CoA acyltransferase, ketoacyl-CoA acyltransferase, ketoacyl-coenzyme A thiolase, long-chain 3-oxoacyl-CoA thiolase, oxoacyl-coenzyme A thiolase, pro-3-ketoacyl-CoA thiolase, 3-oxoadipyl-CoA thiolase, and 3-oxo-5,6-didehydrosuberyl-CoA thiolase. Although there is no particular limitation on classification by EC number, acyltransferases classified into EC 2.3.1.- are preferred, and specific examples include enzymes classified into EC 2.3.1.174, EC 2.3.1.9, EC 2.3.1.16, and EC 2.3.1.223.

For determining whether a protein encoded by a genetic sequence of unknown function corresponds to any of the enzymes described above, BLAST searches can be performed at NCBI (National Center for Biotechnology Information) or other sites to estimate whether the protein corresponds to any of the enzymes.

Preferred and specific examples of these enzymes include enzymes derived from *Acinetobacter* microorganisms such as *Acinetobacter baylyi* and *Acinetobacter radioresistens*; *Aerobacter* microorganisms such as *Aerobacter cloacae; Alcaligenes* microorganisms such as *Alcaligenes faecalis*; *Bacillus* microorganisms such as *Bacillus badius, Bacillus megaterium,* and *Bacillus roseus; Brevibacterium* microorganisms such as *Brevibacterium iodinum; Corynebacterium* microorganisms such as *Corynebacterium acetoacidophilum, Corynebacterium acetoglutamicum, Corynebacterium ammoniagenes,* and *Corynebacterium glutamicum; Cupriavidus* microorganisms such as *Cupriavidus metallidurans, Cupriavidus necator, Cupriavidus numazuensis,* and *Cupriavidus oxalaticus; Delftia* microorganisms such as *Delftia acidovorans; Escherichia* microorganisms such as *Escherichia coli* and *Escherichia fergusonii; Hafnia* microorganisms such as *Hafnia alvei; Microbacterium* microorganisms such as *Microbacterium ammoniaphilum; Nocardioides* microorganisms such as *Nocardioides albus; Planomicrobium* microorganisms such as *Planomicrobium okeanokoites; Pseudomonas* microorganisms such as *Pseudomonas azotoformans, Pseudomonas chlororaphis, Pseudomonas fluorescens, Pseudomonas fragi, Pseudomonas putida, Pseudomonas reptilivora,* and *Pseudomonas taetrolens; Rhizobium* microorganisms such as *Rhizobium radiobacter; Rhodosporidium* microorganisms such as *Rhodosporidium toruloides; Saccharomyces* microorganisms such as *Saccharomyces cerevisiae; Serratia* microorganisms such as *Serratia entomophila, Serratia ficaria, Serratia fonticola, Serratia grimesii, Serratia nematodiphila, Serratia odorifera,* and *Serratia plymuthica; Shimwellia* microorganisms such as *Shimwellia blattae; Sterptomyces* microorganisms such as *Sterptomyces vinaceus, Streptomyces karnatakensis, Streptomyces olivaceus,* and *Streptomyces vinaceus; Yarrowia* microorganisms such as *Yarrowia lipolytica;* and *Yersinia* microorganisms such as *Yersinia ruckeri;* and preferably PaaJ derived from *Escherichia coli* str. K-12 substr. MG1655 (NCBI Protein ID: NP_415915), PcaF derived from *Pseudomonas putida* strain KT2440 (NCBI Protein ID: NP_743536), and PcaF derived from *Corynebacterium glutamicum* strain ATCC13032 (NCBI Protein ID: CAF21057).

In the genetically modified microorganism of the present invention, the activity of enzymes that catalyze the reaction to reduce 3-oxoadipyl-CoA to generate 3-hydroxyadipyl-CoA (reaction B), the reaction to generate 3-hydroxyadipic acid from 3-hydroxyadipyl-CoA (reaction C), and/or the reaction to generate 3-oxoadipic acid from 3-oxoadipyl-CoA (reaction D) is preferably enhanced according to the description in WO2019/107516 and WO2021/187533.

Examples of the enzymes that catalyze the reaction to reduce 3-oxoadipyl-CoA to generate 3-hydroxyadipyl-CoA (reaction B) include enzymes classified into EC 1.1.1.35 as 3-hydroxyacyl-CoA dehydrogenases, and enzymes classified into EC 1.1.1.157 as 3-hydroxybutyryl-CoA dehydrogenase. Specifically, PaaH derived from *Pseudomonas putida* strain KT2440 (NCBI Protein ID: NP_745425.1), PaaH derived from *Escherichia coli* str. K-12 substr. MG1655 (NCBI Protein ID: NP_415913.1), DcaH derived from *Acinetobacter baylyi* strain ADP1 (NCBI Protein ID: CAG68533.1), PaaH derived from *Serratia plymuthica* strain NBRC102599 (NCBI Protein ID: WP_063197120), and a polypeptide derived from *Serratia nematodiphila* strain DSM21420 (NCBI Protein ID: WP_033633399.1) are also included in examples of the enzymes that catalyze the reaction to reduce 3-oxoadipyl-CoA to generate 3-hydroxyadipyl-CoA.

As the enzymes that catalyze the reaction to generate 3-hydroxyadipic acid from 3-hydroxyadipyl-CoA (reaction C) and/or the reaction to generate 3-oxoadipic acid from 3-oxoadipyl-CoA (reaction D), for example, CoA transferase or acetyl-CoA hydrolase can be used, and CoA transferase is preferred.

CoA transferases mean enzymes having a catalytic activity for the reaction to generate carboxylic acid and succinyl-CoA using acyl-CoA and succinic acid as substrates (CoA transferase activity). Examples of CoA transferases, though there is no particular limitation on classification by EC number, include CoA transferases classified into EC 2.8.3.-, CoA transferases classified into EC 2.8.3.6, and acyl-CoA transferases. Specifically, for example, Peal derived from *Pseudomonasputida* strain KT2440 (NCBI Protein ID: NP_746081) and PcaJ (NCBI Protein ID: NP_746082) can be preferably used.

In the present invention, the method to increase the expression level of an organophosphate efflux transporter or to enhance the activity of various enzymes associated with the metabolism of 3HA and/or 3OA is not particularly limited. The methods of increasing the expression levels of polypeptides having various enzyme activities or functions by introducing genes encoding polypeptides having various enzymes or functions into host microorganisms from outside the host microorganisms, increasing the copy numbers of the genes, or modifying the promoter regions or the ribosome-binding sequences upstream of the genes are preferred. These methods may be carried out individually or in combination. The gene to be introduced may be artificially synthesized based on the amino acid sequence of the enzyme present in the database or isolated from natural sources. When artificially synthesizing, the usage frequency of codons corresponding to amino acids may be changed according to the host microorganism to which the gene is introduced. The method to introduce a nucleic acid is not limited to a particular method, and examples of the method that can be used include a method in which the nucleic acid is incorporated in an expression vector capable of autonomous replication in a microorganism is introduced into a host microorganism, and a method in which the nucleic acid is integrated into the genome of a microorganism.

One or more genes may be introduced. Moreover, introduction of a gene and enhancement of the gene expression may be combined.

When a nucleic acid encoding a polypeptide expressed in the present invention is integrated into an expression vector or the genome of a host microorganism, the expression vector or a nucleic acid for genome integration is preferably composed of a promoter, a ribosome-binding sequence, a nucleic acid encoding a polypeptide to be expressed, and a transcription termination sequence. In addition, a gene that controls the activity of the promoter may also be contained.

The promoter used in the present invention is not limited to a particular promoter, provided that the promoter drives the expression of an enzyme in the host microorganism; examples of the promoter include gap promoter, trp promoter, lac promoter, tac promoter, and T7 promoter.

In cases where an expression vector is used in the present invention to introduce nucleic acids or to enhance the expression of polypeptides, the expression vector is not limited to a particular vector, provided that the vector is capable of autonomous replication in the microorganism; examples of the vector include pBBR1MCS vector, pBR322 vector, pMW vector, pET vector, pRSF vector, pCDF vector, pACYC vector, and derivatives of the above vectors.

In cases where a nucleic acid for genome integration is used in the present invention to introduce a nucleic acid or to enhance the expression of a polypeptide, the nucleic acid for genome integration can be introduced by site-specific homologous recombination. The method for site homologous recombination is not limited to a particular method, and examples of the method include a method in which λ Red recombinase and FLP recombinase are used *(*Proc Natl Acad Sci U.S.A., 2000 Jun 6; 97(12): 6640-6645*),* and a method in which λ Red recombinase and the sacB gene are used *(*Biosci Biotechnol Biochem., 2007 Dec; 71(12): 2905-11)*.*

The method of introducing an expression vector or a nucleic acid for genome integration is not limited to a particular method, provided that the method is for introduction of a nucleic acid into a microorganism; examples of the method include the calcium ion method *(*Journal of Molecular Biology, 53,159 (1970*)),* and electroporation *(*NM Calvin, PC Hanawalt. J. Bacteriol, 170 (1988), pp. 2796-2801*).*

Microorganisms originally having an ability to produce 3HA and/or 3OA include the following microorganisms:
the genus *Escherichia,* such as *Escherichia fergusonii* and *Escherichia coli;*
the genus *Serratia,* such as *Serratia grimesii, Serratia ficaria, Serratia fonticola, Serratia odorifera, Serratia plymuthica, Serratia entomophila,* and *Serratia nematodiphila;*
the genus *Pseudomonas,* such as *Pseudomonas chlororaphis, Pseudomonas putida, Pseudomonas azotoformans, Pseudomonas chlororaphis subsp. aureofaciens, Pseudomonas fragi, Pseudomonas fluorescens, Pseudomonas reptilivora,* and *Pseudomonas azotoformans*;
the genus *Hafnia,* such as *Hafnia alvei;*
the genus *Corynebacterium,* such as *Corynebacterium acetoacidophilum, Corynebacterium acetoglutamicum, Corynebacterium ammoniagenes,* and *Corynebacterium glutamicum;*
the genus *Bacillus,* such as *Bacillus badius, Bacillus megaterium,* and *Bacillus roseus;*
the genus *Streptomyces,* such as *Streptomyces vinaceus, Streptomyces karnatakensis,* and *Streptomyces olivaceus;*
the genus *Cupriavidus,* such as *Cupriavidus metallidurans, Cupriavidus necator,* and *Cupriavidus oxalaticus;*
the genus *Acinetobacter,* such as *Acinetobacter baylyi* and *Acinetobacter radioresistens;*
the genus *Alcaligenes,* such as *Alcaligenes faecalis;*
the genus *Nocardioides,* such as *Nocardioides albus;*
the genus *Brevibacterium,* such as *Brevibacterium iodinum;*
the genus *Delftia,* such as *Delftia acidovorans;*
the genus *Shimwellia,* such as *Shimwellia blattae;*
the genus *Aerobacter,* such as *Aerobacter cloacae;*
the genus *Rhizobium,* such as *Rhizobium radiobacter;*
the genus *Planomicrobium,* such as *Planomicrobium okeanokoites;*
the genus *Yarrowia,* such as *Yarrowia lipolytica;*
the genus *Rhodosporidium,* such as *Rhodosporidium toruloides;*
the genus *Microbacterium,* such as *Microbacterium ammoniaphilum;*
the genus *Saccharomyces,* such as *Saccharomyces cerevisiae;* and
the genus *Yersinia,* such as *Yersinia ruckeri.*

Among the microorganisms originally having an ability to produce 3HA and/or 3OA, the microorganisms belonging to the genus *Escherichia* or *Serratia* are preferably used the present invention.

When the genetically modified microorganism of the present invention does not originally have the ability to produce 3HA and/or 3OA, an appropriate combination of nucleic acids encoding the enzymes that catalyze the reactions A, B, and C may be introduced into the microorganism to impart the ability to produce them according to the description in WO2017/209102 and WO2019/107516.

In the present invention, the microorganisms that can be used as hosts to obtain the genetically modified microorganisms are not limited to particular microorganisms, provided that they can be genetically modified, and are preferably microorganisms belonging to the genus *Escherichia, Serratia, Hafnia, Pseudomonas, Corynebacterium, Bacillus, Streptomyces, Cupriavidus, Acinetobacter, Alcaligenes, Brevibacterium, Delftia, Shimwellia, Aerobacter, Rhizobium, Thermobifida, Clostridium, Schizosaccharomyces, Kluyveromyces, Pichia,* or *Candida,* more preferably microorganisms belonging to the genus *Escherichia, Serratia, Hafnia,* or *Pseudomonas,* and particularly preferably microorganisms belonging to the genus *Escherichia* or *Serratia.*

The material for fermentation is a material that can be metabolized by the genetically modified microorganism. The term "metabolize" refers to conversion of a chemical compound, which is taken up by a microorganism from the extracellular environment or is intracellularly generated from a different chemical compound, to another chemical compound through an enzymatic reaction. Sugars can be suitably used as a carbon source. Specific examples of the sugars include monosaccharides, such as glucose, sucrose, fructose, galactose, mannose, xylose, and arabinose; disaccharides and polysaccharides formed by linking these monosaccharides; and saccharified starch solution, molasses, and saccharified solution from cellulose-containing biomass, each containing any of those saccharides.

The above-listed carbon sources may be used individually or in combination, and culturing in a culture medium containing glucose is particularly preferred. When a carbon source is added, the concentration of the carbon source in the culture medium is not particularly limited, and can be appropriately selected depending on the type of the carbon source, or the like. A preferred concentration of glucose is from 5 to 300 g/L.

As the nitrogen source used for culturing the genetically modified microorganism, for example, ammonia gas, aqueous ammonia, ammonium salts, urea, nitric acid salts, other supplementarily used organic nitrogen sources, such as oil cakes, soybean hydrolysate, casein degradation products, other amino acids, vitamins, corn steep liquor, yeast or yeast extract, meat extract, peptides such as peptone, and bacterial cells and hydrolysates of various fermentative bacteria can be used. The concentration of the nitrogen source in the culture medium is not particularly limited, and is preferably from 0.1 to 50 g/L.

As the inorganic salts used for culturing the genetically modified microorganism, for example, phosphoric acid salts, magnesium salts, calcium salts, iron salts, and manganese salts can be appropriately added to the culture medium and used.

The culture conditions for the genetically modified microorganism to produce 3HA and/or 3OA are set by appropriately adjusting or selecting, for example, the culture medium with the above composition, culture temperature, stirring speed, pH, aeration rate, and inoculation amount, depending on the species of the genetically modified microorganism and external conditions, and the like.

The pH range in the culturing is not particularly limited, provided that the genetically modified microorganism can grow, and is preferably from pH5 to 8, more preferably from pH5.5 to 6.8.

The range of the aeration rate condition in the culturing is not particularly limited, provided that 3HA and/or 3OA can be produced, and it is preferred that oxygen remain in the liquid or gas phase in the culture vessel at least at the start of the culturing in order to allow for good growth of the microbial mutant.

In cases where foam is formed in a liquid culture, an antifoaming agent such as a mineral oil, silicone oil, or surfactant may be appropriately added to the culture medium.

After a recoverable amount of 3HA and/or 3OA is produced during culturing of the microorganism, the produced products can be recovered. The produced products can be recovered, for example isolated, according to a commonly used method, in which the culturing is stopped once a product of interest is accumulated to an appropriate level, and the fermentation product is collected from the culture. Specifically, the products can be isolated by column chromatography, ion exchange chromatography, activated charcoal treatment, crystallization, membrane separation, distillation or the like after separating the bacterial cells by centrifugation, filtration or the like. More specifically, examples include, but are not limited to, a method in which an acidic component is added to salts of the products, and the resulting precipitate is collected; a method in which water is removed from the culture by concentration using, for example, a reverse osmosis membrane or an evaporator to increase the concentrations of the products and the products and/or salts of the products are then crystallized and precipitated by cooling or adiabatic crystallization to recover the crystals of the products and/or salts of the products by, for example, centrifugation or filtration; and a method in which an alcohol is added to the culture to produce esters of the products and the resulting esters of the products are subsequently collected by distillation and then hydrolyzed to recover the products. These recovery methods can be appropriately selected and optimized depending on, for example, physical properties of the products.

Adipic acid can be produced by reacting the 3HA and/or 3OA obtained by the present invention with hydrogen (be hydrogenated) in the presence of a hydrogenation catalyst.

The hydrogenation catalyst preferably contains a transition metal element, specifically preferably one or two or more selected from the group consisting of palladium, platinum, ruthenium, rhodium, rhenium, nickel, cobalt, iron, iridium, osmium, copper, and chromium, and more preferably one or two or more selected from the group consisting of palladium, platinum, nickel, cobalt, iron, copper, and chromium.

The hydrogenation catalyst is preferably used with being supported on a carrier from the viewpoints that the amount of metal used can be saved, the active surface of the catalyst is increased, and the like. Supporting the hydrogenation catalyst on a carrier can be carried out according to a known method, such as impregnation, deposition-precipitation, and vapor deposition. Examples of the carrier include carbon, polymer, metallic oxide, metallic sulfide, zeolite, clay, heteropoly acid, solid phosphoric acid, and hydroxyapatite.

Hydrogen to be reacted with 3HA and/or 3OA may be added in a lump or successively. The partial pressure of hydrogen is not particularly limited, but too low partial pressure of hydrogen results in longer reaction time, while too high partial pressure of hydrogen is undesirable for equipment safety. Thus, the partial pressure of hydrogen, at normal temperature, is preferably from 0.1 MPa to 10 MPa (gauge pressure), more preferably from 0.3 MPa to 5 MPa (gauge pressure), and still more preferably from 0.5 MPa to 3 MPa (gauge pressure).

The reaction manner may be one using any of the following reactors: batch tank reactors, semi-batch tank reactors, continuous stirred-tank reactors, continuous plug flow reactors, and trickle-bed tubular reactors. When using a solid hydrogenation catalyst, the reaction can be performed in any of the following manners: suspension, immobilized-bed, moving-bed, and fluidized-bed manners.

The reaction temperature for hydrogenation is not particularly limited, but too low reaction temperatures result in slow reaction rate, and too high reaction temperatures result in high energy consumption, which are thus undesirable. From such a point of view, the reaction temperature is preferably from 100 to 350°C, more preferably from 120 to 300°C, still more preferably from 130 to 280°C, still more preferably from 140 to 250°C, still more preferably from 150 to 230°C, and still more preferably from 160 to 220°C.

The atmosphere in the reactor may contain inert gas, such as nitrogen, helium, and argon, in addition to hydrogen. However, the oxygen concentration is preferably 5 vol% or less because oxygen causes deterioration of hydrogenation catalyst and generation of detonating gas. In addition, from the viewpoint of the stability of 3HA and/or 3OA, and of adipic acid, the amount of ammonia relative to 3HA and/or 3OA is preferably 5 wt% or less, more preferably 3 wt% or less, and still more preferably 0 wt% (i.e., the reaction in the absence of ammonia).

Preferably, 3HA and/or 3OA is/are hydrogenated in the presence of a solvent.

Examples of the solvent that can be used for hydrogenation include methanol, ethanol, n-propanol, isopropanol, n-butanol, isobutanol, tert-butanol, pentane, hexane, cyclohexane, heptane, octane, decane, dimethyl ether, diethyl ether, 1,2-dimethoxyethane, diglyme, tetrahydrofuran, dioxane, methyl acetate, ethyl acetate, n-propyl acetate, n-butyl acetate, γ-butyrolactone, N-methylpyrrolidone, dimethyl sulfoxide, aqueous solvents. A mixed solvent containing two or more of them may be used, but preferably an aqueous solvent is used from the viewpoints of economy and environmental friendliness.

In the present invention, aqueous solvents mean water or mixed solvents mainly containing water mixed with water-miscible organic solvents. The term "mainly containing water" means that the percentage of water in a mixed solvent is more than 50 vol%, preferably 70 vol% or more, and more preferably 90 vol% or more.

Examples of the water-miscible organic solvent include methanol, ethanol, n-propanol, isopropanol, n-butanol, isobutanol, tert-butanol, 1,2-dimethoxyethane, diglyme, tetrahydrofuran, dioxane, γ-butyrolactone, N-methylpyrrolidone, dimethyl sulfoxide, dimethylformamide, dimethylacetamide, and acetone.

The pH of the aqueous solvent is not particularly limited, but is preferably pH2 to 13, more preferably pH3 to 11, and still more preferably pH4 to 10, in consideration of, for example, prevention of catalyst deterioration, prevention of by-product production, and corrosiveness to reaction equipment.

In the case where a solvent other than primary alcohols or secondary alcohols is used as the solvent for hydrogenation, a corresponding adipic acid, adipic acid salt, or adipic acid ester is generated from the carboxylic acid, carboxylic acid salt, carboxylic acid ester of 3-hydroxyadipic acid and/or α-hydromuconic acid. In the case where a solvent containing a primary alcohol or secondary alcohol, such as methanol, ethanol, n-propanol, isopropanol, n-butanol, or isobutanol, is used as the solvent for hydrogenation, a mixture of adipic acid, adipic acid salt, adipic acid monoester, and adipic acid diester is obtained after the reaction. Carboxylic acid, a carboxylic acid salt, a carboxylic acid ester of adipic acid, and a mixture thereof are herein collectively referred to as "adipic acid."

Carboxylic acid of adipic acid obtained in the present invention can be further subjected to esterification reaction to be converted into an adipic acid ester. The esterification method is not particularly limited, and include, for example, dehydration condensation between carboxylic acid and alcohol using an acid catalyst and a condensing agent, and methods using alkylating agents such as diazomethane and alkyl halides.

Adipic acid obtained in the present invention can be isolated and purified by common unit operations such as centrifugation, filtration, membrane filtration, distillation, extraction, crystallization, and dehydration.

Adiponitrile can be produced from adipic acid obtained in the present invention by a known method (e.g., JP 61-24555 B). The obtained adiponitrile can be hydrogenated by a known method (e.g., JP 2000-508305 A) to produce hexamethylenediamine.

Adipic acid obtained in the present invention can be polycondensed with a diamine according to a known method (see, for example, *"*Polyamide Resin Handbook, " Nikkan Kogyo Shimbun, edited by Osamu Fukumoto, January 1998*)* to produce polyamide. Specifically, 1,4-diaminobutane, 1,5-pentanediamine, and hexamethylenediamine as the diamine can be used to produce polyamide 46, polyamide 56, and polyamide 66, respectively.

Polyamide can be processed according to known methods (e.g., WO 2019/208427) to produce polyamide fibers. The thus-obtained polyamide fibers can be used for clothing applications such as innerwear, sportwear, and casual wear, as well as industrial material applications such as airbags and tire cords.

Polyamide can be molded into polyamide molded products according to known methods (e.g., WO 2021/006257). The thus-obtained polyamide molded products can be used for automotive parts, electrical parts, electronic parts, construction parts, various containers, daily necessities, household goods, and sanitary goods.

### EXAMPLES

The present invention will now be specifically described by way of Examples.

### Reference Example 1

Production of a plasmid expressing an enzyme catalyzing a reaction to generate 3OA-CoA and CoA from acetyl-CoA and succinyl-CoA (the reaction A), an enzyme catalyzing a reaction to generate 3HA-CoA from 3OA-CoA (the reaction B), a reaction to generate 3HA from 3HA-CoA (the reaction C), and/or a reaction to generate 3OA from 3OA-CoA (the reaction D)

The pBBR1MCS-2 vector (ME Kovach, (1995), Gene 166: 175-176*)* capable of autonomous replication in E. *coli* and having the kanamycin resistance gene, was cleaved with XhoI to obtain pBBR1MCS-2/XhoI. To integrate a constitutive expression promoter into the vector, primers (SEQ ID NOs: 6 and 7) were designed for use in amplification of an upstream 200-b region (SEQ ID NO: 5) of gapA (NCBI Gene ID: NC_000913.3) using the genomic DNA of *Escherichia coli* str. K-12 substr. MG1655 as a template, and a PCR reaction was performed in accordance with routine procedures. The resulting fragment and the pBBR1MCS-2/XhoI were ligated together using the "In-Fusion HD Cloning Kit" (manufactured by Takara Bio Inc.), and the resulting plasmid was introduced into *E. coli* strain DH5α. The nucleotide sequence on the plasmid extracted from the obtained recombinant *E. coli* strain was confirmed in accordance with routine procedures, and the plasmid was designated as pBBR1MCS-2::Pgap. Then, the pBBR1MCS-2::Pgap was cleaved with Seal to obtain pBBR1MCS-2::Pgap/ScaI. To amplify a gene encoding an enzyme catalyzing the reaction A, primers (SEQ ID NOs: 9 and 10) were designed for use in amplification of the full length of the acyltransferase gene pcaF (NCBI Gene ID: 1041755, SEQ ID NO: 8) using the genomic DNA of *Pseudomonas putida* strain KT2440 as a template, and a PCR reaction was performed in accordance with routine procedures. The resulting fragment and the pBBR1MCS-2::Pgap/ScaI were ligated together using the "In-Fusion HD Cloning Kit" (manufactured by Takara Bio Inc.), and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::AT. Then, the pBBR1MCS-2::AT was cleaved with HpaI to obtain pBBR1MCS-2::AT/HpaI. To amplify a gene encoding an enzyme catalyzing the reaction C and/or the reaction D, primers (SEQ ID NOs: 13 and 14) were designed for use in amplification of a continuous sequence including the full lengths of genes together encoding a CoA transferase, pcaI and pcaJ (NCBI Gene IDs: 1046613 and 1046612; SEQ ID NOs: 11 and 12) using the genomic DNA of *Pseudomonas putida* strain KT2440 as a template, and a PCR reaction was performed in accordance with routine procedures. The resulting fragment and the pBBR1MCS-2::AT/HpaI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::ATCT.

The pBBR1MCS-2::ATCT was cleaved with Seal to obtain pBBR1MCS-2::ATCT/ScaI. To amplify the nucleic acid encoding the polypeptide of SEQ ID NO: 15 that catalyzes the reaction B, primers (SEQ ID NOs: 17 and 18) were designed for use in amplification of the full length of the enzyme gene (SEQ ID NO: 16) that catalyzes the reaction B using the genomic DNA of *Serratia marcescens* strain ATCC13880 as a template, and a PCR reaction was performed in accordance with routine procedures. The resulting fragment and the pBBR1MCS-2::ATCT/ScaI were ligated together using the "In-Fusion HD Cloning Kit" (manufactured by Takara Bio Inc.), and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::ATCTOR.

Next, the kanamycin resistance gene in pBBR1MCS-2::ATCTOR was replaced with the chloramphenicol resistance gene. PCR was performed using pHSG396 (manufactured by Takara Bio Inc.) as a template and the nucleic acids of SEQ ID NOs: 19 and 20 as primers to amplify the chloramphenicol resistance gene. This PCR fragment, and the fragment obtained by cleaving the pBBR1MCS-2::ATCTOR with SapI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBRc::ATCTOR.

### Reference Example 2

### Preparation of plasmid for expression of YhhS

To amplify the gene encoding YhhS and its neighboring regions, PCR was performed using the genomic DNA of *Escherichia coli* str. K-12 substr. MG1655 as a template and the nucleic acids of SEQ ID NOs: 21 and 22 as primers to amplify the nucleic acid comprising the full length of the yhhS gene (SEQ ID NO: 2) and its 0.5-kb upstream and downstream regions. This PCR fragment, and a fragment obtained by cleaving the expression vector pMW119 capable of autonomous replication in *E. coli* (manufactured by Nippon Gene Co., Ltd.) with KpnI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pMW::EcYHHS.

### Reference Example 3

### Preparation of mutant Escherichia microorganism with functional deficiency of YhhS

To delete the function of YhhS in an *Escherichia* microorganism, a mutant *Escherichia* microorganism in which the gene encoding YhhS was deleted was prepared.

The method of deleting the gene of interest was performed according to the method described in Proc Natl Acad Sci USA. 2000 Jun 6; 97(12): 6640-6645*.*

PCR was performed using pKD4 as a template and oligo DNAs of SEQ ID NOs: 23 and 24 as primers to obtain a PCR fragment for yhhS deficiency, having a sequence length of 1.6 kb. pKD46 which is a FRT recombinase expression plasmid, was introduced into *Escherichia coli* str. K-12 substr. BW25113 to obtain an ampicillin-resistant strain. The resulting strain was seeded in 5 mL of LB medium containing 100 µg/mL ampicillin, then incubated at 37°C for 1 day with shaking. Thereafter, 0.5 mL of the culture was seeded in 50 mL of LB medium containing 100 µg/mL ampicillin and 50 mM arabinose, then incubated at 37°C for 2 hours with rotation. The culture was cooled on ice for 20 minutes, and then the bacterial cells were washed with 10%(w/w) glycerol 3 times. The washed pellet was resuspended in 100 µL of 10%(w/w) glycerol, mixed with 5 µL of the PCR fragment, and then cooled in an electroporation cuvette on ice for 10 minutes. Electroporation was performed (3 kV, 200 S2, 25 µF) using "Gene pulser" (manufactured by Bio-Rad Laboratories, Inc.), immediately after which 1 mL of SOC culture medium was added and cultured at 37°C for 2 hours with shaking. The whole culture was applied to LB agar medium containing 25 µg/mL kanamycin, then incubated at 37°C for 1 day. The resulting kanamycin-resistant strain was subjected to colony direct PCR to confirm that the gene of interest was deleted and the kanamycin resistance gene was inserted based on the band length. The primers used were oligo DNAs of SEQ ID NOs: 25 and 26, and 26 and 27, respectively. The resulting mutant *Escherichia* microorganism with functional deficiency of YhhS was designated as EcΔyhhS.

### Reference Example 4

### Preparation of a strain for production of 3HA and/or 3OA with functional deficiency of YhhS and a control strain

The plasmid pBBRc::ATCTOR and pMW119 prepared in Reference Example 1 was introduced to EcΔyhhS prepared in Reference Example 3 to prepare a 3HA- and/or 3OA-producing strain with functional deficiency of YhhS.

EcΔyhhS and *E*. *coli* str. K-12 substr. BW25113 were seeded in 5 mL of LB medium. then incubated with shaking at 37°C for 1 day. Subsequently, 0.5 mL each of the cultures was seeded in 5 mL of LB medium, then incubated at 37°C for 2 hours with shaking. The cultures were cooled on ice for 20 minutes, and then the bacterial cells were washed with 10% (w/w) glycerol 3 times. The washed pellets were resuspended in 100 µL of 10% (w/w) glycerol, mixed with 1 µL of pBBRc::ATCTOR and pMW119, and then cooled in an electroporation cuvette on ice for 10 minutes. Electroporation was performed (3 kV, 200 S2, 25 µF) using "Gene pulser" (manufactured by Bio-Rad Laboratories, Inc.), immediately after which 1 mL of SOC culture medium was added and cultured at 37°C for 1 hour with shaking. Subsequently, 50 µL each of the cultures was applied to LB agar medium containing 15 µg/mL chloramphenicol and 100 µg/mL ampicillin, then incubated at 37°C for 1 day. The resulting strain was designated as EcΔyhhS/3HA_pMW.

pBBRc::ATCTOR and pMW119 were introduced to an *Escherichia coli* str. K-12 substr. BW25113 by the method described above to obtain a 3HA and/or 3OA-producing strain without functional deficiency of YhhS (EcWT/3HA_pMW) as a control strain.

### Comparative Example 1

### Test for production of 3HA by a control strain

A loopful of the control strain (EcWT/3HA_pMW) was inoculated into 5 mL (ϕ 18-mm glass test tube, aluminum plug) of the culture medium I (10 g/L Bacto Tryptone (manufactured by Difco Laboratories), 5 g/L Bacto Yeast Extract (manufactured by Difco Laboratories), 5 g/L sodium chloride, 15 µg/mL chloramphenicol, 100 µg/mL ampicillin) adjusted to pH 7, then incubated at 30°C for 24 hours with shaking at 120 min⁻¹. Subsequently, 0.25 mL of the culture fluid was added to 5 mL (ϕ18-mm glass test tube, aluminum plug) of the culture medium II (50 g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto Tryptone, 1.25 g/L Bacto Yeast Extract, 15 µg/mL chloramphenicol, 100 µg/mL ampicillin) adjusted to pH 6.5, and statically incubated at 30°C.

The supernatant separated from bacterial cells by centrifugation of the obtained culture fluid was processed by membrane treatment using "Millex-GV" (0.22 µm; PVDF; manufactured by Merck), and the resulting filtrate was analyzed by LC-MS/MS under the following conditions to measure the concentrations of 3HA and other products accumulated in the culture supernatant, and of sugars remaining in the culture medium without being used. Additionally, the yields of 3HA calculated based on the results according to the following Formula (2) are presented in Table 1.

### Quantitative analyses of 3HA by LC-MS/MS

•HPLC: 1290 Infinity (manufactured by Agilent Technologies, Inc.)
   Column: Synergi hydro-RP (manufactured by Phenomenex Inc.), length: 100 mm, internal diameter: 3 mm, particle size: 2.5 µm
   Mobile phase: 0.1% aqueous formic acid solution / methanol = 70/30
   Flow rate: 0.3 mL/min
   Column temperature: 40°C
   LC detector: 1260DAD VL+ (210 nm)
·MS/MS: Triple-Quad LC/MS (manufactured by Agilent Technologies, Inc.)
   Ionization method: ESI in negative mode.

### Quantitative analyses of sugars by HPLC

·HPLC: Shimadzu Prominence (manufactured by Shimadzu Corporation)
Column: Shodex Sugar SH1011 (manufactured by Showa Denko K.K.), length: 300 mm, internal diameter: 8 mm, particle size: 6 µm
Mobile phase: 0.05 M aqueous sulfuric acid solution
Flow rate: 0.6 mL/min
Column temperature: 65°C
Detector: RID-10A (manufactured by Shimadzu Corporation) Yield of 3HA (%) = amount of produced 3HA (mol)/amount of consumed sugar (mol) × 100

### Reference Example 5

### Test for production of 3HA by a strain for production of 3HA and/or 3OA with functional deficiency of YhhS

A test for production of 3HA was performed using the strain for production of 3HA and/or 3OA with functional deficiency of YhhS prepared in Reference Example 4 (EcΔyhhS/3HA_pMW) in the same manner as in Comparative Example 1. The results are shown in Table 1.

Comparing the results of Comparative Example 1 and Reference Example 5, the strain for production of 3HA and/or 3OA with functional deficiency of YhhS (Reference Example 5) showed lower 3HA yield than the control strain without functional deficiency of YhhS (Comparative Example 1). These results and the previous finding that YhhS has an organophosphate efflux function suggest that YhhS contributes to the improvement of the 3HA productivity by having an organophosphate efflux function.

### Reference Example 6

### Preparation of strain for production of 3HA and/or 3OA with restored YhhS function

The plasmids pBBRc::ATCTOR and pMW::EcYHHS prepared in Reference Examples 1 and 2 were introduced to EcΔyhhS with functional deficiency of YhhS in the same manner as in Reference Example 4 to prepare a strain for production of 3HA and/or 3OA with restored YhhS function (EcΔyhhS/3HA_EcYHHS).

### Reference Example 7

### Test for production of 3HA by a strain for production of 3HA and/or 3OA with restored YhhS function

In order to further confirm that YhhS has 3HA efflux function, a test for production of 3HA was performed using the strain for production of 3HA and/or 3OA with restored YhhS function prepared in Reference Example 6 (EcΔyhhS/3HA_EcYHHS) in the same manner as in Comparative Example 1. The results are shown in Table 1.

Comparing the results of Reference Example 5 and Reference Example 7, the strain for production of 3HA and/or 3OA with restored YhhS function (Reference Example 7) showed higher 3HA yield than the strain for production of 3HA and/or 3OA with functional deficiency of YhhS (Reference Example 5), further suggesting that YhhS contributes to the improvement of the 3HA productivity by having a 3HA efflux function.

### Example 1

### Preparation of strain for production of 3HA and/or 3OA with increased YhhS expression

The plasmids pBBRc::ATCTOR and pMW::EcYHHS prepared in Reference Examples 1 and 2 were introduced to *E. coli* str. K-12 substr. BW25113 in the same manner as in Reference Example 4 to prepare a strain for production of 3HA and/or 3OA with increased YhhS expression (EcWT/3HA_EcYHHS).

### Example 2

### Test for production of 3HA by a strain for production of 3HA and/or 3OA with increased YhhS expression

In order to confirm that the increase in the YhhS expression has an effect of improving the 3HA productivity, a test for production of 3HA was performed using the strain for production of 3HA and/or 3OA with increased YhhS expression (EcWT/3HA_EcYHHS) prepared in Example 1 in the same manner as in Comparative Example 1. The results are shown in Table 1.

Comparing the results of Comparative Example 1 and Example 2, the strain for production of 3HA and/or 3OA with increased YhhS expression (Example 2) showed improved 3HA yield as compared to the control strain (Comparative Example 1). Therefore, it is suggested that the increase of the YhhS expression leads to enhanced 3HA efflux function, thereby improving the 3HA productivity.

**[Table 1]**

| | Strain | 3HA yield (%) |
|---|---|---|
| Comparative Example 1 | EcWT/3HA_pMW | 1.65 |
| Reference Example 5 | EcΔyhhS/3HA_pMW | 1.19 |
| Reference Example 7 | EcΔyhhS/3HA_EcYHHS | 1.47 |
| Example 2 | EcWT/3HA_EcYHHS | 1.86 |

### Comparative Example 2

### Test for production of 3OA by a control strain

A loopful of the control strain (EcWT/3HA_pMW) was inoculated into 5 mL of the culture medium I (ϕ18-mm glass test tube, aluminum plug) adjusted to pH7, then incubated at 30°C for 24 hours with shaking at 120min⁻¹. Subsequently, 0.25 mL of the culture fluid was added to 5 mL (ϕ18-mm glass test tube, aluminum plug) of the culture medium III (10 g/L glucose, 10 g/L sodium succinate, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto Tryptone, 1.25 g/L Bacto Yeast Extract, 15 µg/mL chloramphenicol, 100 µg/mL ampicillin) adjusted to pH 6.5, then incubated at 30°C with shaking.

The supernatant separated from bacterial cells by centrifugation of the obtained culture fluid was analyzed in the same manner as in Comparative Example 1 to measure the concentrations of 3OA and other products accumulated in the culture supernatant, and of sugars remaining in the culture medium without being used. Additionally, the yields of 3OA calculated based on the results according to the following Formula (3) are presented in Table 2. Yield of 3OA (%) = amount of produced 3OA (mol)/amount of consumed sugar (mol) × 100

### Example 3

### Test for production of 3OA by a strain for production of 3HA and/or 3OA with increased YhhS expression

In order to confirm that the increase in the YhhS expression has an effect of improving the 3OA productivity, a test for production of 3OA was performed using the strain for production of 3HA and/or 3OA with increased YhhS expression (EcWT/3HA_EcYHHS) prepared in Example 1 in the same manner as in Comparative Example 2. The results are shown in Table 2.

Comparing the results of Comparative Example 2 and Example 3, the strain for production of 3HA and/or 3OA with increased YhhS expression (Example 3) showed improved 3OA yield as compared to the control strain (Comparative Example 2). Therefore, it is suggested that the increase of the YhhS expression leads to enhanced 3OA efflux function, thereby improving the 3OA productivity.

**[Table 2]**

| | Strain | 3OA yield (%) |
|---|---|---|
| Comparative Example 2 | EcWT/3HA_pMW | 2.82 |
| Example 3 | EcWT/3HA_EcYHHS | 3.11 |

### Reference Example 8

### Preparation of plasmids for expression of various transporters

To amplify the upstream region comprising the promoter and the downstream region comprising the terminator of the yhhS gene of *E. coli,* the genomic DNA of *Escherichia coli* str. K-12 substr. MG1655 was used as a template to amplify the 0.5-kb upstream region and the 0.5-kb downstream region of the yhhS gene. The nucleic acids of SEQ ID NOs: 34 and 35, and 36 and 37 were used as primers, respectively. This PCR fragment, and a fragment obtained by cleaving the expression vector pMW119 capable of autonomous replication in *E. coli* (manufactured by Nippon Gene Co., Ltd.) with SacI and KpnI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pMW::Pro-Ter.

### Reference Example 9

### Preparation of plasmid for expression of YhhS homolog

For expression of YhhS homolog, a yhhS homolog sequence of *Salmonella enterica* strain ATCC9150 was genetically synthesized, and then the entire length of the gene was amplified using the sequence as a template and the nucleic acids of SEQ ID NOs: 38 and 39 as primers. This PCR fragment, and the fragment obtained by cleaving the pMW::Pro-Ter with SacI and KpnI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pMW::SeYHHS.

Similarly, a yhhS homolog sequence of *Klebsiella pneumoniae* strain MGH78578 was genetically synthesized, and then the entire length of the gene was amplified using the sequence as a template and the nucleic acids of SEQ ID NOs: 40 and 41 as primers. This PCR fragment was inserted into pMW::Pro-Ter in the same manner as in preparation of pMW::SeYHHS, and the obtained plasmid was designated as pMW::KpYHHS.

Similarly, a yhhS homolog sequence of *Pectobacterium carotovorum* strain PC1 was genetically synthesized, and then the entire length of the gene was amplified using the sequence as a template and the nucleic acids of SEQ ID NOs: 42 and 43 as primers. This PCR fragment was inserted into pMW::Pro-Ter in the same manner as in preparation of pMW::SeYHHS, and the obtained plasmid was designated as pMW::PcYHHS.

Similarly, using the genomic DNA of *Serratia grimesii* strain NBRC13537 as a template and the nucleic acids of SEQ ID NOs: 44 and 45 as primers, the entire length of the gene was amplified. This PCR fragment was inserted into pMW::Pro-Ter in the same manner as in preparation of pMW::SeYHHS, and the obtained plasmid was designated as pMW::SgYHHS.

### Example 4

### Preparation of strain for production of 3HA and/or 3OA with increased expression of YhhS homolog

The plasmid pBBRc::ATCTOR prepared in Reference Example 1 was introduced into *E. coli* str. K-12 substr. BW25113 in the same manner as in Reference Example 4. The plasmid pMW::SeYHHS, pMW::KpYHHS, pMW::PcYHHS, or pMW::SgYHHS prepared in Reference Example 9 was introduced to the resulting strain to prepare strains for production of 3HA and/or 3OA with increased expression of a YhhS homolog (EcWT/3HA_SeYHHS, EcWT/3HA_KpYHHS, EcWT/3HA _PcYHHS, and EcWT/3HA_SgYHHS).

### Example 5

### Test for production of 3HA by a strain for production of 3HA and/or 3OA with increased expression of YhhS homolog

In order to confirm that the increase in the expression of a YhhS homolog has an effect of improving the 3HA productivity, a test for production of 3HA was performed using the strains for production of 3HA and/or 3OA with increased expression of a YhhS homolog (EcWT/3HA_SeYHHS, EcWT/3HA_KpYHHS, EcWT/3HA_PcYHHS, EcWT/3HA_SgYHHS) prepared in Example 4 in the same manner as in Comparative Example 1. The results are shown in Table 3.

Comparing the results of Comparative Example 1 and Example 5, the strains for production of 3HA and/or 3OA with increased expression of a YhhS homolog (Example 5) showed improved 3HA yields as compared to the control strain (Comparative Example 1). Therefore, it is suggested that the increase of the expression of a YhhS homolog leads to enhanced 3HA efflux function, thereby improving the 3HA productivity.

**[Table 3]**

| | Strain | 3HA yield (%) |
|---|---|---|
| Comparative Example 1 | EcWT/3HA_pMW | 1.65 |
| Example 5 | EcWT/3HA_SeYHHS | 2.09 |
| Example 5 | EcWT/3HA_KpYHHS | 1.87 |
| Example 5 | EcWT/3HA_PcYHHS | 1.93 |
| Example 5 | EcWT/3HA_SgYHHS | 1.92 |

### Example 6

### Test for production of 3OA by a strain for production of 3HA and/or 3OA with increased expression of YhhS homolog

In order to confirm that the increase in the expression of a YhhS homolog has an effect of improving the 3OA productivity, a test for production of 3OA was performed using the strains for production of 3HA and/or 3OA with increased expression of a YhhS homolog (EcWT/3HA_SeYHHS, EcWT/3HA_KpYHHS, EcWT/3HA_PcYHHS, EcWT/3HA_SgYHHS) prepared in Example 4 in the same manner as in Comparative Example 2. The results are shown in Table 4.

Comparing the results of Comparative Example 2 and Example 6, the strains for production of 3HA and/or 3OA with increased expression of a YhhS homolog (Example 6) showed improved 3OA yields as compared to the control strain (Comparative Example 2). Therefore, it is suggested that the increase of the expression of a YhhS homolog leads to enhanced 3OA efflux function, thereby improving the 3OA productivity.

**Table 4]**

| | Strain | 3OA yield (%) |
|---|---|---|
| Comparative Example 2 | EcWT/3HA_pMW | 2.82 |
| Example 6 | EcWT/3HA_SeYHHS | 3.11 |
| Example 6 | EcWT/3HA_KpYHHS | 3.29 |
| Example 6 | EcWT/3HA_PcYHHS | 4.70 |
| Example 6 | EcWT/3HA_SgYHHS | 3.30 |

### Reference Example 10

### Preparation of plasmid for expression of organophosphate efflux transporter

To allow expression of an organophosphate efflux transporter MdtD, using the genomic DNA of *Escherichia coli* str. K-12 substr. MG1655 as a template and the nucleic acids of SEQ ID NOs: 52 and 53 as primers, the entire length of the gene was amplified. This PCR fragment was inserted into pMW::Pro-Ter in the same manner as in preparation of pMW::SeYHHS, and the obtained plasmid was designated as pMW::EcMDTD.

Similarly, to allow expression of RhtB, using the genomic DNA of *Escherichia coli* str. K-12 substr. MG1655 as a template and the nucleic acids of SEQ ID NOs: 54 and 55 as primers, the entire length of the gene was amplified. This PCR fragment was inserted into pMW::Pro-Ter in the same manner as in preparation of pMW::SeYHHS, and the obtained plasmid was designated as pMW::EcRHTB.

Similarly, to allow expression of MFS40, using the artificially-synthesized MFS40 gene of *Aspergillus oryzae* strain RIB40 as a template and the nucleic acids of SEQ ID NOs: 56 and 57 as primers, the entire length of the gene was amplified. This PCR fragment was inserted into pMW::Pro-Ter in the same manner as in preparation of pMW::SeYHHS, and the obtained plasmid was designated as pMW::AoMFS40.

### Example 7

### Preparation of strain for production of 3HA and/or 3OA with increased expression of organophosphate efflux transporter

The plasmid pBBRc::ATCTOR prepared in Reference Example 1 was introduced into *E. coli* str. K-12 substr. BW25113 in the same manner as in Reference Example 4. The plasmid pMW::EcMDTD, pMW::EcRHTB, or pMW::AoMFS40 prepared in Reference Example 10 was introduced to the resulting strain to prepare strains for production of 3HA and/or 3OA with increased expression of an organophosphate efflux transporter (EcWT/3HA_EcMDTD, EcWT/3HA_EcRHTB, and EcWT/3HA _AoMFS40).

### Example 8

### Test for production of 3HA by strain for production of 3HA and/or 3OA with increased expression of organophosphate efflux transporter

In order to confirm that the increase in the expression of an organophosphate efflux transporter has an effect of improving the 3HA productivity, a test for production of 3HA was performed using the strains for production of 3HA and/or 3OA with increased expression of an organophosphate efflux transporter (EcWT/3HA_EcMDTD, EcWT/3HA_EcRHTB, and EcWT/3HA_AoMFS40) prepared in Example 7 in the same manner as in Comparative Example 1. The results are shown in Table 5.

Comparing the results of Comparative Example 1 and Example 8, the strains for production of 3HA and/or 3OA with increased expression of an organophosphate efflux transporter (Example 8) showed improved 3HA yield as compared to the control strain (Comparative Example 1). Therefore, it is suggested that the increase of the expression of an organophosphate efflux transporter leads to enhanced 3HA efflux function, thereby improving the 3HA productivity.

**[Table 5]**

| | Strain | 3HA yield (%) |
|---|---|---|
| Comparative Example 1 | EcWT/3HA_pMW | 1.65 |
| Example 8 | EcWT/3HA_EcMDTD | 2.01 |
| Example 8 | EcWT/3HA_EcRHTB | 1.86 |
| Example 8 | EcWT/3HA_AoMFS40 | 1.79 |

### Reference Example 11

### Preparation of strain for production of 3HA and/or 3OA with increased expression of protein that belongs to MFS and does not have organophosphate efflux function

YhhS, MdtD, and MFS40 are all proteins belonging to MFS. In this Reference Example, to test whether a protein that belongs to MFS and does not have organophosphate efflux function has a 3HA efflux function, a strain for production of 3HA and/or 3OA with increased expression of EmrD was prepared.

EmrD is a protein belonging to MFS and called Multidrug resistance protein D. A specific example thereof is EmrD derived from *Escherichia coli* str. K-12 substr. MG1655 (NCBI Protein ID: NP_418129, SEQ ID NO: 58), and an example of the gene encoding EmrD is emrD derived from *Escherichia coli* str. K-12 substr. MG1655 (NCBI Gene ID: 948180, SEQ ID NO: 59). It is described in JP 2017-528126 A that EmrD does not have efflux function for an organophosphate compound O-phosphoserine.

To allow expression of EmrD, using the genomic DNA of *Escherichia coli* str. K-12 substr. MG1655 as a template and the nucleic acids of SEQ ID NOs: 60 and 61 as primers, the entire length of the gene was amplified. This PCR fragment was inserted into pMW::Pro-Ter in the same manner as in preparation of pMW::SeYHHS, and the obtained plasmid was designated as pMW::EcEMRD.

### Reference Example 12

### Preparation of strain for production of 3HA and/or 3OA with increased expression of protein that belongs to MFS and does not have organophosphate efflux function

The plasmid pBBRc::ATCTOR prepared in Reference Example 1 was introduced into *E. coli* str. K-12 substr. BW25113 in the same manner as in Reference Example 4. The plasmid pMW::EcEMRD prepared in Reference Example 11 was introduced to the resulting strain to prepare a strain for production of 3HA and/or 3OA with increased expression of a protein belonging to MFS and not having any organophosphate efflux function (EcWT/3HA_pMW::EcEMRD).

### Reference Example 13

### Test for production of 3HA by strain for production of 3HA and/or 3OA with increased expression of protein that belongs to MFS and does not have organophosphate efflux function

A test for production of 3HA was performed using the strain for production of 3HA and/or 3OA with increased expression of a protein that belongs to MFS and does not have an organophosphate efflux function prepared in Reference Example 12 (EcWT/3HA_pMW::EcEMRD) in the same manner as in Comparative Example 1. The results are shown in Table 6.

Comparing the results of Comparative Example 1 and Reference Example 13, the strains for production of 3HA and/or 3OA with increased expression of an organophosphate efflux transporter (Reference Example 13) showed reduced 3HA yield as compared to the control strain (Comparative Example 1). Therefore, it is suggested that the increase of the expression of a protein that does not have an organophosphate efflux function does not lead to enhancement of the 3HA efflux function, so that the 3HA productivity is not improved.

**[Table 6]**

| | Strain | 3HAyield (%) |
|---|---|---|
| Comparative Example 1 | EcWT/3HA_pMW | 1.65 |
| Reference Example 13 | EcWT/3HA_EcEMRD | 1.45 |

## Claims

1. A genetically modified microorganism having an ability to produce 3-hydroxyadipic acid and/or 3-oxoadipic acid,
wherein the activity of an enzyme that catalyzes the reaction to generate 3-oxoadipyl-CoA and CoA from succinyl-CoA and acetyl-CoA is enhanced, and
wherein the expression level of an organophosphate efflux transporter is increased.

2. The genetically modified microorganism of claim 1, wherein the organophosphate efflux transporter is a phosphoserine efflux transporter and/or a glyphosate efflux transporter.

3. The genetically modified microorganism of claim 1 or 2, wherein the organophosphate efflux transporter is YhhS, MdtD, RhtB, MFS40, or a homolog thereof.

4. The genetically modified microorganism of any one of claims 1 to 3, wherein the expression level of the organophosphate efflux transporter is increased by introducing a gene encoding any of the polypeptides (A) to (C) below:
(A) a polypeptide consisting of the amino acid sequence of SEQ ID NO: 1, 3, 28-30, 46, 48, or 50;
(B) a polypeptide having a sequence identity of 60% or more to the amino acid sequence of SEQ ID NO: 1, 3, 28-30, 46, 48, or 50, and having a function to improve the productivity of 3-hydroxyadipic acid and/or 3-oxoadipic acid; and
(C) a polypeptide consisting of the same amino acid sequence as the amino acid sequence of SEQ ID NO: 1, 3, 28-30, 46, 48, or 50 except that one or several amino acids are replaced, deleted, inserted and/or added, and having a function to improve the productivity of 3-hydroxyadipic acid and/or 3-oxoadipic acid.

5. The genetically modified microorganism of any one of claims 1 to 4, further having enhanced activities of enzymes that catalyze the reaction to reduce 3-oxoadipyl-CoA to generate 3-hydroxyadipyl-CoA and/or the reaction to generate 3-hydroxyadipic acid from 3-hydroxyadipyl-CoA.

6. The genetically modified microorganism of any one of claims 1 to 5, wherein activity of an enzyme that catalyzes the reaction to generate 3-oxoadipic acid from 3-oxoadipyl-CoA is further enhanced.

7. The genetically modified microorganism of any one of claims 1 to 6, wherein the microorganism is a microorganism belonging to the genus *Escherichia* or *Serratia.*

8. A method of producing 3-hydroxyadipic acid and/or 3-oxoadipic acid, comprising the step of culturing the genetically modified microorganism of any one of claims 1 to 7.

9. A method of producing a genetically modified microorganism, comprising the steps of:
genetically modifying a microorganism having an ability to produce 3-hydroxyadipic acid and/or 3-oxoadipic acid to enhance the activity of an enzyme that catalyzes the reaction to generate 3-oxoadipyl-CoA and CoA from succinyl-CoA and acetyl-CoA; and
increasing the expression level of YhhS or a homolog thereof in the microorganism.

10. A method of producing 3-hydroxyadipic acid and/or 3-oxoadipic acid, comprising the step of culturing a microorganism having an ability to produce 3-hydroxyadipic acid and/or 3-oxoadipic acid and having increased expression level of an organophosphate efflux transporter.

11. A method of producing adipic acid, comprising:
the step A of producing 3-hydroxyadipic acid and/or 3-oxoadipic acid by the method of claim 8 or 10; and
the step B (hydrogenation step) of reacting the 3-hydroxyadipic acid and/or 3-oxoadipic acid with hydrogen in the presence of a hydrogenation catalyst.

12. A method of producing a polyamide, comprising:
the step of producing adipic acid according to the method of claim 11, and
the step C of polycondensing the adipic acid and a diamine.

13. The method of producing a polyamide of claim 11, wherein the diamine is a diamine containing 1,4-butanediamine, 1,5-pentanediamine, or hexamethylenediamine.
